(19) Europäisches Patentamt
European Patent Office
Office européen des brevets

(11) Publication number: 0 385 712 B1

(12) # EUROPEAN PATENT SPECIFICATION

(45) Date of publication of patent specification: **12.01.94 Bulletin 94/02**

(51) Int. Cl.⁵: **A61K 37/30**, C07K 7/10, C07K 99/42, C07K 99/46

(21) Application number: **90302055.0**

(22) Date of filing: **27.02.90**

(54) **A peptide for use in the manufacture of a composition for prevention or treatment of a condition susceptible to treatment with a platelet aggregation inhibition agent.**

(30) Priority: **28.02.89 JP 49265/89**

(43) Date of publication of application: **05.09.90 Bulletin 90/36**

(45) Publication of the grant of the patent: **12.01.94 Bulletin 94/02**

(84) Designated Contracting States: **DE ES FR GB IT**

(56) References cited:
**EP-A- 0 300 737**
**WO-A-85/01658**
**US-A- 4 530 838**
**CHEMICAL ABSTRACTS, vol. 109, no. 25, 19 December 1988, page 128, abstract no. 223002x, Columbus, Ohio, US; A. OEHLEN et al.: "Calcitonin gene-related peptide desensitizes skeletal muscle arterioles to substance P in vivo"**
**CHEMICAL ABSTRACTS, vol. 111, no. 7, 14 August 1989, page 132, abstract no. 50993g, Columbus, Ohio, US; A. OEHLEN et al.: "Substance P activates leukocytes and platelets in rabbit microvessels"**

(73) Proprietor: **ASAHI KASEI KOGYO KABUSHIKI KAISHA**
**2-6, Dojimahama 1-chome Kita-ku**
**Osaka (JP)**

(72) Inventor: **Noda, Toshiharu**
**908-5 Kosaka, Izunagaoko-cho**
**Tagata-gun, Shizuoka (JP)**
Inventor: **Ueda, Seigo**
**6-4-21 Akatsuka**
**Itabashi-ku, Tokyo (JP)**
Inventor: **Matsumoto, Yoshihisa**
**2-23-2 Senkawa**
**Toshima-ku, Tokyo (JP)**
Inventor: **Matsushita, Satoru**
**2-15-9 Nakakokubu**
**Ichikawa-shi, Chiba (JP)**

(74) Representative: **Woods, Geoffrey Corlett et al**
**J.A. KEMP & CO. 14 South Square Gray's Inn**
**London WC1R 5LX (GB)**

EP 0 385 712 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the use of a peptide in the manufacture of a platelet aggregation inhibition medicament.

Human CGRP (hereinafter sometimes referred to as h-CGRP) is a peptide which is known to act on bone metabolism and the central nervous system. (Nature, 308(19): 746-748 (1984), FEBS Letters, 183(2): 403 (1985), Neuropeptides, 4: 425-434 (1984) and Nature, 313(3): 54-56 (1984).) Porcine CGRP (hereinafter sometimes referred to as p-CGRP) is known to increase the heart rate {Neuropeptides, 9: 75-82 (1987)). Rat CGRP (hereinafter sometimes referred to as r-CGRP) is known to have vasodilation and gastric acid secretion inhibitory action (British J. Pharmacol., 86: 544 (1985) and Regulatory Peptides, 12: 81-89 (1985)). Furthermore human CGRP derivatives, chicken CGRP (hereinafter sometimes referred to as c-CGRP) and c-CGRP derivatives are known to have serum calcium and phosphorus reducing action (JP-A-62-129297, JP-A-63-126894, JP-A-63-258490 and JP-A-64-26598).

The amino acid sequence of CGRP is:

```
                                          1
                                       H - A -

        ┌────────── S ──────────── S ──────────┐
        │2      3      4       5        6      7│
- C y s - B - T h r - A l a - T h r - C y s

   8       9        10       11       12
- V a l - T h r - H i s - A r g - L e u

   13     14  15     16       17      18
- A l a - C - D - L e u - S e r - A r g

   19       20       21      22  23    24
- S e r - G l y - G l y - E - F - L y s

  25   26       27       28       29       30
- G - A s n - P h e - V a l - P r o - T h r

  31   32       33       34     35   36
- I - V a l - G l y - S e r - J - A l a
```

37
-Phe-$NH_2$

h-α-CGRP (A=Ala, B=Asp, C=Gly, D=Leu, E=Val, F=Val, G=Asn, I=Asn, J=Lys)

h-β-CGRP (A=Ala, B=Asn, C=Gly, D=Leu, E=Met, F=Val, G=Ser, I=Asn, J=Lys)

r-α-CGRP (A=Ser, B=Asn, C=Gly, D=Leu, E=Val, F=Val, G=Asp, I=Asn, J=Glu)

r-β-CGRP (A=Ser, B=Asn, C=Gly, D=Leu, E=Val, F=Val, G=Asp, I=Asn, J=Lys)

p-CGRP (A=Ser, B=Asn, C=Gly, D=Leu, E=Met, F=Val, G=Ser, I=Asp, J=Glu)

c-CGRP (A=Ala, B=Asn, C=Asp, D=Phe, E=Val, F=Gly, G=Asn, I=Asn, J=Lys)

We have surprisingly found that CGRP has an activity on human platelet aggregation inhibition and is consequently able to prevent and/or treat conditions susceptible to treatment with a platelet aggregation inhibition agent, for example arteriosclerosis or thrombosis.

Accordingly the present invention provides the use of a calcitonin gene related peptide (CGRP) or a derivative thereof having platelet aggregation inhibitory activity, or a salt thereof, in the manufacture of a platelet aggregation inhibition medicament.

The medicament may be used to prevent or treat a condition susceptible to treatment with a platelet aggregation inhibition agent. The condition may be, for example, arteriosclerosis or thrombosis.

CGRP and its derivatives, or a salt thereof, can be synthesized by known methods for peptide synthesis, for example liquid phase or solid phase synthesis.

Examples of CGRP are h -α- CGRP, h -β- CGRP, c - CGRP, r - α- CGRP, r - β- CGRP and p-CGRP.

Examples of derivatives of CGRP are desalanyl-deamino-h-α-CGRP, desalanyl-deamino- h-β-CGRP, desalanyl - [$Asu^{2,7}$]-h-α-CGRP, desalanyl - [$Asu^{2,7}$] - h-β-CGRP, [$Asn^3$, $Phe^{15}$, $Gly^{23}$] - h-α-CGRP, desalanyl-deamino - [$Asn^3$, $Phe^{15}$, $Gly^{23}$] -h- α-CGRP, [$Asn^{3,}$ $Asp^{14}$, $Gly^{23}$] -h -α-CGRP, desalanyl-deamino-[$Asn^3$, $Asp^{14}$, $Gly^{23}$] - h-α-CGRP, [$Asn^{3'}$ $Asp^{14}$, $Phe^{15}$] - h- α-CGRP, desalanyl-deamino - [$Asn^3$, $Asp^{14}$, $Phe^{15}$] - h- α-CGRP, [$Asp^{14}$] - h-α-CGRP, desalanyl-deamino - [$Asp^{14}$ ] -h- α-CGRP, [$Asn^3$, [$Glu^{14}$, $Gly^{23}$] - h-α-CGRP, desalanyl-deamino - [$Asn^3$, $Glu^{14}$, $Gly^{23}$] -h - α-CGRP, [$Asn^3$, $Glu^{14}$, $Phe^{15}$] - h - α-CGRP, desalanyl-deamino- [$Asn^3$, [$Glu^{14}$, $Phe^{15}$] -h - α-CGRP, [$Glu^{14}$ ] -h - α - CGRP, desalanyl-deamino- [$Glu^{14}$] - h-α- CGRP, desalanyl-[$Asu^{2,\ 7}$] -c-CGRP, desalanyl - [$Asp^{3,}$ $Asu^{2,\ 7}$] -c-CGRP and desalanyl-deamino-c-CGRP.

The salts are pharmacologically non-toxic salts. Examples are salts of an inorganic acid such as the hydrochloride and sulfate and of an organic acid such as the acetate, tartrate, succinate and malate.

The medicament is preferably administered parenterally, for example by intravenous, intramuscular, subcutaneous, intranasal, percutaneous, intravaginal or per anum administration. The dose depends on the active principle, and is preferably 500 ~1 μg/day/man for parenteral administration.

The medicament is preferably in a form suitable for parenteral administration. The medicament may be in, for example, injectable form, rectal suppository, percutaneous form or intranasal form. The formulations are not limited. The formulations depend on the form of the medicament. For example, injectable forms are prepared by dissolving the active principle in water for injection with buffering agent, isotonic agent, pH adjusting agent and stabilizing agent, passed through a disinfection filter and packed into ampules. Alternatively the active principle is dissolved in water for injection together with extender and stabilizer, sterilized by passing through a disinfection filter to prepare a germ free mixture, packed in vials and freeze dried to prepare the formulation for injection. A rectal suppository can be prepared, for example, by dissolving or suspending the active principle with absorption accelerator having chelating activity such as sodium pectinate or sodium arginate and isotonic agent such as sodium chloride or glucose in distilled water or an oily vehicle. A nasal administration form can be prepared by adding antiseptics such as benzalkonium chloride and filling up with an aseptic gas.

The formulations, for example a spray form, nasal administration form, percutaneous tape formulation or ointment form can also be prepared by known methods.

The acute toxicity and platelet aggregation inhibition activity of CGRP and derivatives thereof are illustrated below:

(1) Acute toxicity :

A solution of each of the following compounds in 0.1 M aqueous sodium acetate containing 0.1 % bovine serum albumin is administered into a rat, Wister strain, body weight 80 - 90 g, 80 μg/kg, intraveneously in the tail. No deaths were observed.

h - α - CGRP,
h - β- CGRP,
c - CGRP,
r - α - CGRP,
r - β- CGRP,
p - CGRP,
desalanyl-deamino-h-α-CGRP,
desalanyl-deamino-h-β-CGRP,
desalanyl- [$Asu^{2,7}$] -h - α-CGRP,
desalanyl- [$Asu^{2,7}$] -h -β-CGRP,
[$Asn^3$, $Phe^{15}$, $Gly^{23}$] -h -α-CGRP,
desalanyl-deamino- [$Asn^3$, $Phe^{15}$, $Gly^{23}$] -h -α-CGRP,
[$Asn^3$, $Asp^{14}$, $Gly^{23}$] -h -α-CGRP,
desalanyl-deamino- [$Asn^3$, $Asp^{14}$, $Gly^{23}$] -h -α-CGRP,
[$Asn^3$, $Asp^{14}$, $Phe^{15}$] -h -α-CGRP,
desalanyl-deamino- [$Asn^3$, $Asp^{14}$, $Phe^{15}$] -h -α-CGRP,
[$Asp^{14}$ ] -h -α-CGRP,
desalanyl-deamino- [$Asp^{14}$ ] -h -α-CGRP,
[$Asn^3$, $Glu^{14}$, $Gly^{23}$] -h -α-CGRP,
desalanyl-deamino- [$Asn^3$, $Glu^{14}$, $Gly^{23}$] -h -α-CGRP,

[$Asn^3$, $Glu^{14}$, $Phe^{15}$] -h -α-CGRP,
desalanyl-deamino- [$Asn^3$, $Glu^{14}$, $Phe^{15}$] -h -α-CGRP,
[$Glu^{14}$ ] -h -α-CGRP,
desalanyl-deamino- [$Glu^{14}$ ] -h -α-CGRP,
desalanyl- [$Asu^{2,7}$] - c-CGRP,
desalanyl- [$Asp^{3,}$ $Asu^{2,7}$)-c-CGRP and
desalanyl-deamino-c-CGRP.

(2) Inhibitory action on human platelet aggregation :

The maximum aggregation ratios of collagen solution, 1.0 μg/mℓ, 20 μℓ, or epinephrine solution, 0.125 μmole/mℓ, 20μℓ , when added to human platelets, 4 ×$10^4$ particles/200μℓ, are 73 % and 55%, respectively. Under the same conditions, a sample of CGRPs 20μℓ and collagen solution 20μℓ or epinephrine 20μℓ, both of which have the same concentration as above, is added to platelets, 4 × $10^4$ particles/200 μℓ , and the maximum aggregation ratio is measured. The aggregation inhibition ratio is calculated by the following equations:

| Aggregation agent | Maximum aggregation ratio ( % ) |
|---|---|
| Collagen | $\frac{73 - \text{Maxumum aggregation ratio}}{73} \times 100$ |
| Epinephrine | $\frac{55 - \text{Maximum aggregation ratio}}{55} \times 100$ |

The result are shown in Table 1:

| Sample(Final concentration in reaction mixture) | Aggregation agent | Aggregation ratio |
|---|---|---|
| h - CGRP | | |
| $5 \times 10^{-7}$M | collagen | 27 % |
| $5 \times 10^{-9}$M | collagen | 19 % |
| $5 \times 10^{-6}$M | epinephrine | 76 % |
| desalanyl-deamino-c-CGRP | | |
| $5 \times 10^{-6}$M | collagen | 82 % |

Almost the same levels of activity as h-CGRP are observed when the platelet aggregation inhibitory activities of samples other than those in Table 1 are measured.

The active principle has an inhibitory action on platelet aggregation, and hence is useful for use in a pharmaceutical composition for prevention and/or treatment of conditions susceptible to treatment with a platelet aggregation inhibition agent, such as arteriosclerosis or thrombosis.

## Examples

The following Examples further illustrate the present invention.

### Example 1

h-α-CGRP 60 mg, mannitol 10g and stabilizing agent dissolved in water for injection 2 l, are passed through a disinfection filter, packed in vials, each 1 mℓ/vial, and freeze dried to prepare an injectable formulation of h-α-CGRP, 30 μg/vial. The formulation is administered after dissolving in physiological saline.

### Example 2

h-α-CGRP 60 mg was dissolved in water for injection 6 l, containing buffer solution, isotonic solution, pH adjusting agent and stabilizing agent, passed through a disinfection filter, packed in ampules, each 1 mℓ/ampule, and sealed to prepare an injectable formulation of h-α-CGRP, 10 μg/ampule.

### Example 3

Desalanyl-deamino-c-CGRP 60 mg, mannitol 10g and stabilizing agent were dissolved in water for injection 2 l, passed through a disinfection filter, packed in vials, each 1 m ℓ/vial, and freeze dried to prepare an injectable formulation of desalanyl-deamino-c-CGRP, each 30 μg/vial.

The formulation is administered after dissolving in physiological saline.

Example 4

Desalanyl-deamino-c-CGRP 60 mg was dissolved in water for injection 6 l, containing buffer solution, isotonic solution, pH adjustering agent and stabilizing agent, passed through disinfection filter, packed in ampules, each 1 m ℓ/ampule, and sealed to prepare an injectable formulation of desalanyl-deamino -c-CGRP, 10 μg/ampule.

Injectable formulations of CGRP other than the above can also be prepared according to the same procedures as above.

Example 5

h-α-CGRP 100 mg and D-mannitol 400 mg were dissolved in distilled water 24 mℓ. A solution thereof was freezedried and pulverized in a mortar to obtain a homogenized dried powder containing h-α-CGRP 0.2 mg/mg. This powder 20 mg and D-glucuronic acid 100 mg were mixed well in a mortar, dextran (Sigma Chem. Co., mean molecular weight 40200) 800 mg, was gradually added therein, then mixed to prepare a homoginized powder formulation. The powder is provided as a formulation for intranasal administration.

Example 6

Desalanyl-deamino-c-CGRP 10 mg and D-mannitol 40 mg were dissolved in distilled water 5 mℓ. A solution thereof was freeze dried to obtain a homogenized freeze dried powder containing desalanyl-deamino-c-CGRP 0.2 mg/mg.

Succinic acid 500 mg and D-mannitol 500 mg were dissolved in distilled water 100 m ℓ and freeze dried to obtain a freeze dried powder. The powder 10 mg and the powder 10 mg containing desalanyl-deamino-c-CGRP were mixed well in a mortar, and hydroxypropyl cellulose (Nihon Soda Co. HPC-L) 440 mg was gradually added thereto and mixed to prepare a homogenized powder formulation. This powder formulation is provided in nasal administration form.

## Claims

1. Use of a calcitonin gene related peptide (CGRP) or a derivative thereof having platelet aggregation inhibitory activity, or a salt thereof, in the manufacture of a platelet aggregation inhibition medicament.

2. Use according to claim 1 wherein the CGRP is a human α-CGRP, human β-CGRP, chicken CGRP, rat α-CGRP, rat β-CGRP or porcine CGRP.

3. Use according to claim 2 wherein the CGRP is desalanyl-deamino-human-α-CGRP, desalanyl-deamino-human β-CGRP, desalanyl-[$Asu^{2,7}$]-human α-CGRP, desalanyl-[$Asu^{2,7}$]-human β-CGRP, [$Asn^3$, $Phe^{15}$, $Gly^{23}$]-human α-CGRP, desalanyl-deamino-[$Asn^3$, $Phe^{15}$, $Gly^{23}$]-human α-GCRP, [$Asn^3$, $Asp^{14}$, $Gly^{23}$]-human α-CGRP, desalanyl-deamino-[$Asn^3$, $Asp^{14}$, $Gly^{23}$]-human α-CGRP, [$Asn^3$, $Asp^{14}$, $Phe^{15}$]-human α-CGRP, desalanyl-deamino- [$Asn^3$, $Asp^{14}$, $Phe^{15}$]-human α-CGRP, [$Asp^{14}$]-human α-CGRP, desalanyl-deamino-[$Asp^{14}$]-human α-CGRP, [$Asn^3$, $Glu^{14}$, $Gly^{23}$]-human α-CGRP, desalanyl-deamino[$Asn^3$, $Glu^{14}$, $Gly^{23}$]-human α-CGRP, [$Asn^3$, $Glu^{14}$, $Phe^{15}$]-human α-CGRP, desalanyl-deamino-[$Asn^3$, $Glu^{14}$. $Phe^{15}$]-human α-CGRP, [$Glu^{14}$]-human α-CGRP, desalanyl-deamino-[$Glu^{14}$]-human α-CGRP, desalanyl - [$Asu^{2,7}$]-chicken-CGRP or desalanyl deamino-chicken-CGRP.

4. Use according to any one of the preceding claims wherein the medicament is for the prevention or treatment of arteriosclerosis or thrombosis.

5. Use according to any one of the preceding claims wherein the CGRP is in the form of a hydrochloride, sulfate, acetate, tartrate, succinate or malate salt.

6. Use according to any one of the preceding claims wherein the medicament is in a form suitable for parenteral administration.

## Patentansprüche

1. Verwendung eines von einem Calcitoningen abgeleiteten Peptids (CGRP) oder eines Derivats davon mit Thrombozyten-Aggregationshemmung oder eines Salzes davon zur Herstellung eines Medikaments zur Thrombozyten-Aggregationshemmung.

2. Verwendung nach Anspruch 1, dadurch **gekennzeichnet,** daß das CGRP ein menschliches α-CGRP, ein menschliches β-CGRP, ein Hühner-CGRP, ein Ratten-α-CGRP, ein Ratten-β-CGRP oder ein Schweine-CGRP ist.

3. Verwendung nach Anspruch 2, dadurch **gekennzeichnet**, daß das CGRP menschliches Desalanyl-desamino-α-CGRP, menschliches Desalanyl-desamino-β-CGRP, menschliches Desalanyl-[$Asu^{2,7}$]-α-CGRP, menschliches Desalanyl-[$Asu^{2,7}$]-β-CGRP, menschliches [$Asn^3$, $Phe^{15}$, $Gly^{23}$]-α-CGRP, menschliches Desalanyl-desamino-[$Asn^3$, $Phe^{15}$, $Gly^{23}$ ]-α-CGRP, menschliches [$Asn^3$, $Asp^{14}$, $Gly^{23}$]-α-CGRP, menschliches Desalanyl-desamino-[$Asn^3$, $Asp^{14}$, $Gly^{23}$]-α-CGRP, menschliches [$Asn^3$, $Asp^{14}$, $Phe^{15}$]-α-CGRP, menschliches Desalanyl-desamino-[$Asn^3$, $Asp^{14}$, $Phe^{15}$]-α-CGRP, menschliches [$Asp^{14}$]-α-CGRP, menschliches Desalanyl-desamino-[$Asp^{14}$]-α-CGRP, menschliches [$Asn^3$, $Glu^{14}$, $Gly^{23}$]-α-CGRP, menschliches Desalanyl-desamino-[$Asn^3$, $Glu^{14}$, $Gly^{23}$]-α-CGRP, menschliches [$Asn^3$, $Glu^{14}$, $Phe^{15}$]-α-CGRP, menschliches Desalanyl-desamino-[$Asn^3$, $Glu^{14}$, $Phe^{15}$]-α-CGRP, menschliches [$Glu^{14}$]-α-CGRP, menschliches Desalanyl-desamino-[$Glu^{14}$]-α-CGRP, Hühner-Desalanyl-[$Asu^{2,7}$]-CGRP oder Hühner-Desalanyl-desamino-CGRP ist.

4. Verwendung nach einem der vorausgehenden Ansprüche, dadurch **gekennzeichnet**, daß das Medikament der Verhütung oder Behandlung von Arteriosklerose oder Thrombose dient.

5. Verwendung nach einem der vorausgehenden Ansprüche, dadurch **gekennzeichnet**, daß das CGRP in der Form eines Hydrochlorid-, Sulfat-, Acetat-, Tartrat-, Succinat- oder Malatsalzes vorliegt.

6. Verwendung nach einem der vorausgehenden Ansprüche, dadurch **gekennzeichnet**, daß das Medikament in einer Form, die für die parenterale Verabreichung geeignet ist, vorliegt.

## Revendications

1. Utilisation d'un peptide relié à un gène de calcitonine (CGRP) ou un dérivé de celui-ci ayant une activité d'inhibition d'agrégation plaquettaire, ou un sel de celui-ci, dans la fabrication d'un médicament pour l'inhibition de l'agrégation plaquettaire.

2. Utilisation selon la revendication 1, dans laquelle le CGRP est un α-CGRP humain, un β-CGRP humain, un CGRP du poulet, un α-CGRP du rat, un β-CGRP du rat ou un CGRP porcin.

3. Utilisation selon la revendication 2, dans laquelle le CGRP est le désalanyl-déamino-α-CGRP humain, désalanyl-déamino-β-CGRP humain, désalanyl-[$Asu^{2,7}$]-α-CGRP humain, désalanyl [$Asu^{2,7}$]-β-CGRP humain, [$Asn^3$, $Phe^{15}$, $Gly^{23}$]-α-CGRP humain, désalanyl-déamino-[$Asn^3$, $Phe^{15}$, $Gly^{23}$]-α-CGRP humain, [$Asn^3$, $Asp^{14}$, $Gly^{23}$]-α-CGRP humain, désalanyl-déamino-[$Asn^3$, $Asp^{14}$, $Gly^{23}$]-α-CGRP humain, [$Asn^3$, $Asp^{14}$, $Phe^{15}$]-α-CGRP humain, désalanyl déamino- [$Asn^3$, $Asp^{14}$, $Phe^{15}$]-α-CGRP humain, [$Asp^{14}$]-α-CGRP humain, désalanyl-déamino-[$Asp^{14}$]-α-CGRP humain, [$Asn^3$, $Glu^{14}$, $Gly^{23}$]-α-CGRP humain, désalanyl-déamino [$Asn^3$, $Glu^{14}$, $Gly^{23}$]-α-CGRP humain, [$Asn^3$, $Glu^{14}$, $Phe^{15}$]-α-CGRP humain, désalanyl-déamino-[$Asn^3$, $Glu^{14}$, $Phe^{15}$]-α-CGRP humain, [$Glu^{14}$]-α-CGRP humain, désalanyl-déamino-[$Glu^{14}$]-α-CGRP humain, désalanyl-[$Asu^{2,7}$]-CGRP du poulet ou désalanyl déamino - CGRP du poulet.

4. Utilisation selon l'une quelconque des revendications précédentes dans laquelle le médicament est pour la prévention ou le traitement de l'artériosclérose ou de la trombose.

5. Utilisation selon l'une quelconque des revendications précédentes dans laquelle le CGRP est sous la forme d'un sel hydrochlorure, sulfate, acétate, tartrate, succinate ou malate.

6. Utilisation selon l'une quelconque des revendications précédentes dans laquelle le médicament est sous la forme appropriée pour une administration parentérale.